# EUROPÄISCHE PATENTSCHRIFT

(11) **EP 2 482 868 B1**
(45) Veröffentlichungstag und Bekanntmachung des Hinweises auf die Patenterteilung: **03.09.2014**
(21) Anmeldenummer: 10765374.3
(22) Anmeldetag: 23.09.2010
(51) Int. Cl.: A61M 1/16

(54) **VORRICHTUNG ZUR DIALYSEBEHANDLUNG UND VERFAHREN ZUM BILANZIEREN VON FRISCHER UND VERBRAUCHTER DIALYSIERFLÜSSIGKEIT**
DEVICE FOR DIALYSIS TREATMENT AND METHOD FOR BALANCING FRESH AND USED DIALYSIS LIQUID
DISPOSITIF DE TRAITEMENT PAR DIALYSE ET PROCÉDÉ D'ÉQUILIBRAGE DU LIQUIDE DE DIALYSE FRAIS ET USAGÉ

(30) Priorität: 29.09.2009 DE 102009043284; 07.10.2009 DE 102009048561
(43) Veröffentlichungstag der Anmeldung: 08.08.2012
(73) Patentinhaber: Fresenius Medical Care Deutschland GmbH, 61352 Bad Homburg v.d.H. (DE)
(72) Erfinder: WEIS, Manfred, 66606 St. Wendel (DE); LAUER, Martin, 66606 St. Wendel (DE); KREBER, Stefan, 66113 Saarbrücken (DE)
(74) Vertreter: Oppermann, Frank
(86) Internationale Anmeldenummer: PCT/EP2010/005826
(87) Internationale Veröffentlichungsnummer: WO 2011/038858

(56) Entgegenhaltungen:
- EP-A1- 0 687 474
- EP-A1- 0 867 195
- EP-A1- 1 393 761
- DE-C1- 19 830 928
- US-A1- 2005 000 868

## Beschreibung

Die Erfindung betrifft eine Vorrichtung zur Dialysebehandlung, die über Mittel zum Bilanzieren von frischer und verbrauchter Dialysierflüssigkeit verfügt. Darüber hinaus betrifft die Erfindung ein Verfahren zum Bilanzieren von frischer und verbrauchter Dialysierflüssigkeit.

Zur Entfernung von harnpflichtigen Substanzen und zum Flüssigkeitsentzug werden beim chronischen Nierenversagen verschiedene Verfahren zur apparativen Blutreinigung bzw. Blutbehandlung eingesetzt. Bei der Hämodialyse (HD) überwiegt der diffusive Stofftransport, während bei der Hämofiltration (HF) ein konvektiver Stofftransport über die semipermeable Membran des Dialysators bzw. Filters stattfindet. Eine Kombination aus beiden Verfahren ist die Hämodiafiltration (HDF). Wenn im Folgenden von einem Dialysator die Rede ist, wird unter einem Dialysator auch ein Filter verstanden.

Wegen der großen Austauschmengen besteht bei dem oben genannten Verfahren die Notwendigkeit der exakten Bilanzierung von der dem Dialysator zugeführten frischen Dialysierflüssigkeit und der aus dem Dialysator abgeführten verbrauchten Dialysierflüssigkeit unter Berücksichtigung der über die Membran des Dialysators dem Patienten entzogenen Menge an Flüssigkeit über die gesamte Behandlungszeit. Zum Stand der Technik gehören gravimetrische und volumetrische Bilanziersysteme. Die dem Patienten durch Ultrafiltration entzogene Menge an Flüssigkeit kann dem Patienten bei kontrollierter Entwässerung teilweise oder vollständig wieder als pyrogenfreies Dialysat oder Substituat zugeführt werden. Die Zufuhr von pyrogenfreiem Dialysat oder Substituat im extrakorporalen Blutkreislauf stromauf des Dialysators wird als Prädilution und die Zufuhr von Dialysat oder Substituat stromab des Dialysators als Postdilution bezeichnet. Beide Verfahren können auch miteinander kombiniert werden.

Die DE-A-28 38 414 beschreibt eine Dialysevorrichtung mit einem Bilanziersystem, das über zwei Bilanzkammern verfügt, deren Bilanzkammerhälften wechselweise mit frischer Dialysierflüssigeit unter Verwerfung verbrauchter Dialysierflüssigkeit aus der jeweils anderen Kammerhälfte befüllt werden. Der zwischen dem Bilanziersystem und dem Dialysator eingeschlossene Teil des Flüssigkeitskreislaufs verhält sich daher wie ein geschlossenes System, dessen Volumen konstant ist. Über eine Ultrafiltrationspumpe kann dem geschlossenen System Flüssigkeit entzogen werden. Bei einem Flüssigkeitsentzug entsteht an der semipermeablen Membran des Dialysators ein Druckgradient, der dazu führt, dass dem Patienten dieselbe Menge an Flüssigkeit entzogen wird, wie dem geschlossenen System mit der Ultrafiltrationspumpe Flüssigkeit entzogen wird.

Für die Dialysebehandlung ist von großer Bedeutung, dass die dem Patient durch Ultrafiltration entzogene Menge an Flüssigkeit genau bestimmbar ist. Daher werden an die Genauigkeit der Bilanzierung sehr hohe Anforderungen gestellt. Dies setzt aber voraus, dass mit den Bilanzkammern der Bilanziersysteme eine exakte Bilanzierung von frischer und verbrauchter Dialysierflüssigkeit möglich ist. Denn schon geringe Fehler bei der Bilanzierung in den aufeinanderfolgenden Arbeitstakten des Bilanziersystems können sich über die gesamte Behandlungsdauer zu nicht mehr tolerierbaren Fehlbilanzierungen akkumulieren.

Es sind volumetrische Bilanziersysteme bekannt, bei denen frische bzw. verbrauchte Dialysierflüssigkeit in die bzw. aus den Bilanzkammern mit Pumpen gefördert werden. Es sind aber auch Bilanziersysteme bekannt, bei denen von einer Membran in zwei Kammerhälften unterteilte Bilanzkammern mit einem Arbeitsfluid beaufschlagt werden, um frische bzw. verbrauchte Dialysierflüssigkeit zu fördern.

Die DE-A-198 30 928 beschreibt eine Dialysevorrichtung mit einem volumetrischen Bilanziersystem, dessen Bilanzkammern als ein zur einmaligen Verwendung bestimmtes Disposable ausgebildet sind. Das Bilanziersystem umfasst insgesamt vier Bilanzkammern, die von einer Membran jeweils in zwei Bilanzkammerhälften unterteilt sind. Eine der beiden Bilanzkammerhälften wird zur Förderung von frischer oder verbrauchter Dialysierflüssigkeit, die sich in der jeweils anderen Kammerhälfte befindet, mit einem Arbeitsfluid beaufschlagt. Die vier Bilanzkammern bilden ein erstes Paar von Bilanzkammern, die zur Förderung von frischer Dialysierflüssigkeit bestimmt sind, und ein zweites Paar von Bilanzkammern, die zur Förderung von verbrauchter Dialysierflüssigkeit bestimmt sind. Die frische Dialysierflüssigkeit wird von einer Dialysierflüssigkeitsquelle mit den beiden Bilanzkammern des einen Paars von Bilanzkammern zu der Dialysierflüssigkeitskammer des Dialysators gefördert. Die verbrauchte Dialysierflüssikeit aus der Dialysierflüssigkeitskammer des Dialysators wird mit den beiden Bilanzkammern des anderen Paars von Bilanzkammern zu einem Auslass gefördert. Dabei werden die jeweiligen Bilanzkammerhälften der Bilanzkammern wechselweise mit frischer bzw. verbrauchter Dialysierflüssigkeit befüllt, so dass die Dialysierflüssigkeit kontinuierlich durch die Dialysierflüssigkeitskammer des Dialysators strömt.

Um die jeweiligen Bilanzkammern wechselweise mit frischer und verbrauchter Dialysierflüssigkeit füllen zu können, sind die zugehörigen Bilanzkammerhälften in die von der Dialysierflüssigkeitsquelle zu der Dialysierflüssigkeitskammer des Dialysators führende Zuführleitung für frische Dialysierflüssigkeit bzw. in die von der Dialysierflüssigkeitskammer des Dialysators abgehende und zu dem Auslass führende Abführleitung für verbrauchte Dialysierflüssigkeit geschaltet. In den jeweiligen Zweigen der Zuführ- und Abführleitung befinden sich Absparorgane, um die Flüssigkeitsströmung steuern zu können. Die gesamte Strömungsführung und Steuerung ist weder dazu bestimmt noch geeignet, die zur Förderung frischer Dialysierflüssigkeit bestimmten Bilanzkammern des einen Paars von Bilanzkammern zur Förderung von verbrauchter Dialysierflüssigkeit einzusetzen und umgekehrt.

Nachteilig ist, dass eine exakte Bilanzierung von frischer und verbrauchter Dialysierflüssigkeit voraussetzt, dass die Volumina der einzelnen Bilanzkammerhälften exakt gleich sind. Die stellt aber besonders hohe Anforderungen an die Fertigungstoleranzen, die insbesondere bei einer als Disposable ausgebildeten Anordnung von Bilanzkammern nur mit einem hohen technischen Aufwand einzuhalten sind.

Aus der US 2005/0000868 A1 ist ebenfalls ein Bilanziersystem bekannt, dass über vier Bilanzklammern verfügt, mit denen dem Patienten zuzuführendes Substituat und über die Membran des Dialysators entzogenes Filtrat gegeneinander bilanziert werden. Auch bei diesem Bilanziersystem bilden jeweils zwei Kammern ein Paar von Kammern, die entweder Substituat oder Filtrat aufnehmen. Zum Befüllen und Entleeren der Kammern dienen Pumpen in den Zuführ- und Abführleitungen. Daher brauchen die Kammern selbst nicht durch eine Membran in zwei Kammerhälften unterteilt zu werden.

Der Erfindung liegt die Aufgabe zugrunde, eine Vorrichtung zur Dialysebehandlung zu schaffen, die eine exakte Bilanzierung von frischer und verbrauchter Dialysierflüssigkeit erlaubt. Darüber hinaus ist eine Aufgabe der Erfindung, ein Verfahren zur exakten Bilanzierung von frischer und verbrauchter Dialysierflüssigkeit bereit zu stellen.

Die Lösung dieser Aufgaben erfolgt erfindungsgemäß mit den Merkmalen der Patentansprüche 1 und 10. Vorteilhafte Ausführungsformen der Erfindung sind Gegenstand der Unteransprüche.

Die erfindungsgemäße Vorrichtung zur Dialysebehandlung und das erfindungsgemäße Verfahren zum Bilanzieren von frischer und verbrauchter Dialysierflüssigkeit zeichnen sich dadurch aus, dass die einzelnen Bilanzkammern des Bilanziersystems sowohl frische Dialysierflüssigkeit als auch verbrauchte Dialysierflüssigkeit aufnehmen, wobei die Funktionen der Kammern wechselweise vertauscht werden. Dadurch kann eine exakte Bilanzierung selbst dann erreicht werden, wenn sich die Volumina der einzelnen Kammern voneinander unterscheiden. Über die gesamte Behandlungsdauer gleichen sich die Abweichungen der Volumina wegen des zyklischen Vertauschens der Kammern gegenseitig aus.

Für die Erfindung ist unerheblich, wie frische und verbrauchte Dialysierflüssigkeit den einzelnen Bilanzkammern zugeführt bzw. frische und verbrauchte Dialysierflüssigkeit von den einzelnen Bilanzkammern abgeführt wird.

Bei der erfindungsgemäßen Vorrichtung zur Dialysebehandlung sind die Mittel zum Leiten von frischer und verbrauchter Dialysierflüssigkeit, bei denen es sich vorzugsweise um Schlauchleitungen handelt, derart ausgebildet, dass frische Dialysierflüssigkeit aus einer Dialysierflüssigkeitsquelle jeder Bilanzkammer zugeführt werden kann und frische Dialysierflüssigkeit aus jeder Bilanzkammer der Dialysierflüssigkeitskammer des Dialysators geführt werden kann, und verbrauchte Dialysierflüssigkeit aus der Dialysierflüssigkeitskammer des Dialysators jeder Bilanzkammer zugeführt werden kann und verbrauchte Dialysierflüssigkeit aus jeder Bilanzkammer abgeführt werden kann. Die einzelnen Bilanzkammern werden in aufeinanderfolgenden Arbeitstakten jeweils mit frischer oder verbrauchter Dialysierflüssigkeit gefüllt bzw. die mit frischer oder verbrauchter Dialysierflüssigkeit gefüllten Bilanzkammern werden entleert. Die vier Bilanzkammern bilden zwei Paare von Bilanzkammern, wobei in jedem Arbeitstakt aus einer Bilanzkammer des einen Paars von Bilanzkammern der Dialysierflüssigkeitskammer frische Dialysierflüssigkeit zugeführt und der anderen Bilanzkammer des einen Paars von Bilanzkammern verbrauchte Dialysierflüssigkeit aus der Dialysierflüssigkeitskammer zugeführt wird, während einer Bilanzkammer von dem anderen Paar von Bilanzkammern von der Dialysierflüssigkeitsquelle frische Dialysierflüssigkeit zugeführt und aus der anderen Bilanzkammer von dem anderen Paar von Bilanzkammer verbrauchte Dialysierflüssigkeit abgeführt wird. In den aufeinanderfolgenden Arbeitstakten werden die Funktionen der einzelnen Bilanzkammern gegenseitig vertauscht, so dass sich Abweichungen in den Volumina der Bilanzkammern über die gesamte Behandlungsdauer kompensieren können.

Für die Erfindung ist unerheblich, wie die Dialysierflüssigkeit in die bzw. aus den Bilanzkammern gefördert wird. Bei einer bevorzugten Ausführungsform der Erfindung werden die Bilanzkammern zum Fördern von frischer bzw. verbrauchter Dialysierflüssigkeit mit einem Überdruck oder einem Unterdruck beaufschlagt, mit der frische bzw. verbrauchte Dialysierflüssigkeit aus der Kammer gedrückt oder in die Kammer gesaugt werden kann. Die Mittel zum Fördern von Dialysierflüssigkeit weisen weiterhin vorzugsweise Mittel zum Öffnen und Absperren der Mittel zum Leiten von Dialysierflüssigkeit, vorzugsweise Mittel zum Öffnen und Absperren der Schlauchleitungen auf, um die Flüssigkeitsströmung steuern zu können.

Es ist grundsätzlich aber auch möglich, zur Förderung von frischer und verbrauchter Dialysierflüssigkeit mit den Bilanzkammern anstelle von Luft ein Fluid zu verwenden, wie aus dem Stand der Technik bekannt ist. Dann ist es aber erforderlich, die beiden Bilanzkammern mit einer Membran in zwei Bilanzkammerhälften zu unterteilen, wobei die eine Bilanzklammerhälfte zur Aufnahme von frischer und verbrauchter Dialysierflüssigkeit und die andere Bilanzkammerhälfte zur Aufnahme des Arbeitsfluids dient. Weiterhin ist es auch möglich, anstelle von Druckluft oder einem Arbeitsfluid die Dialysierflüssigkeit mit Pumpen zu fördern, wie aus dem Stand der Technik bekannt ist.

Da jeweils zwei Bilanzkammern miteinander in Verbindung stehen, ist es-grundsätzlich möglich, nur in einer der beiden Bilanzkammern einen Überdruck zu erzeugen, während die andere Bilanzkammer entlüftet wird. Andererseits ist es aber auch möglich, in nur einer der beiden Bilanzkammern einen Unterdruck zu erzeugen, während die andere Bilanzkammer belüftet wird. Vorzugsweise wird in der einen Bilanzkammer ein Überdruck und in der anderen Bilanzkammer ein Unterdruck erzeugt.

Bei einer weiteren bevorzugten Ausführungsform weisen die Mittel zum Fördern von Dialysierflüssigkeit Mittel zum Messen des Füllstandes der Bilanzkammern auf, wobei die Steuereinheit derart ausgebildet ist, dass die Mittel zum Öffnen und Absperren in Abhängigkeit von einem vorgegebenen Wert für den Füllstand in den Bilanzkammern geöffnet oder geschlossen werden. Dadurch wird erreicht, dass bei einem bestimmten Füllstand die Flüssigkeitsströmung unterbrochen wird. Dieser Füllstand braucht nicht dem maximalen Füllvolumen der Bilanzkammern zu entsprechen. Für eine exakte Bilanzierung von frischer bzw. verbrauchter Dialysierflüssigkeit, bei der die Menge an frischer Dialysierflüssigkeit exakt der Menge an verbrauchter Dialysierflüssigkeit entsprechen soll, wird für sämtliche Bilanzkammern der gleiche Wert für den Füllstand vorgegeben.

Eine besonders bevorzugte Ausführungsform sieht die Möglichkeit vor, dem Patienten über die Behandlungsdauer Flüssigkeit zuzuführen bzw. Flüssigkeit zu entziehen. Bei dieser Ausführungsform werden zwei unterschiedliche Werte für den Füllstand der Bilanzkammem vorgegeben. Dabei wird die eine Bilanzkammer des einen Paars von Bilanzkammern, von der frische Dialysierflüssigkeit der Dialysierflüssigkeitskammer zugeführt wird, bis zu dem ersten Wert für den Füllstand befüllt, während die andere Bilanzkammer des einen Paars von Bilanzkammern, der verbrauchte Dialysierflüssigkeit aus der Dialysierflüssigkeitskammer zugeführt wird, bis zu einem zweiten Wert für den Füllstand befüllt, wobei der erste Wert von dem zweiten Wert verschieden ist. Entsprechend wird die eine Bilanzkammer von dem anderen Paar von Bilanzkammern, der frische Dialysierflüssigkeit von der Dialysierflüssigkeitsquelle zugeführt wird, bis zu dem ersten Wert für den Füllstand befüllt, während die andere Bilanzkammer von dem anderen Paar von Bilanzkammern, von der verbrauchte Dialysierflüssigkeit aus der Dialysierflüssigkeitskammer abgeführt wird, bis zu dem zweiten Wert für den Füllstand befüllt wird. Die Differenz zwischen dem ersten und dem zweiten Wert entspricht der Menge an Flüssigkeit, die über die Membran des Dialysators dem Patienten entzogen oder dem Patienten zugeführt wird. Wenn der erste Wert für den Füllstand kleiner als der zweite Wert für den Füllstand ist, wird dem Blut des Patienten Filtrat entzogen. Hierbei können unterschiedliche Werte für den Füllstand in einem oder mehreren oder sämtlichen Arbeitstakten eines Zyklus vorgegeben werden, wobei nur eine, zwei, drei oder vier Kammern unterschiedlich befüllt werden. Die Ultrafiltrationsmenge ergibt sich dann aus der Summe der Füllstandsdifferenzen der beteiligten Bilanzkammern. Die auf Grund von Herstellungstolleranzen oder Messfehlern sich möglicherweise ergebenden Abweichungen in den einzelnen Differenzvolumen von dem gewünschten Wert der vom behandelnden Arzt einzustellenden Ultrafiltrationsrate sind wegen der vergleichsweise kleinen Füllstandsdifferenzen wesentlich kleiner als die Abweichungen, die sich in einem normalen Betrieb der Bilanzkammern ergeben.

Bei einer weiteren bevorzugten Ausführungsform sind die Mittel zum Bilanzieren von frischer und verbrauchter Dialysierflüssigkeit als zur einmaligen Verwendung bestimmtes Disposable ausgebildet. Dadurch wird die Ausrüstung der Dialysevorrichtung mit fest verbauten Bilanzkammern überflüssig. Darüber hinaus entfällt die aufwendige Sterilisierung der ansonsten in der Maschine verbleibenden flüssigkeitsführenden Teile. Die Ausbildung eines Bilanzsystems als Disposable ist dem Fachmann als solches bekannt. Ein derartiges Disposable ist beispielsweise aus der DE 198 30 928 Clund der DE 195 46 028 C2 bekannt.

Im Folgenden werden zwei Ausführungsbeispiele der Erfindung unter Bezugnahme auf die Zeichnungen näher erläutert.

Es zeigen:
- Fig. 1: Eine Vorrichtung zur Dialyse in stark vereinfachter schematischer Darstellung, wobei der erste Arbeitstakt eines Arbeitszyklus dargestellt ist.
- Fig. 2: die Dialysevorrichtung von Fig. 1, wobei der zweite Arbeitstakt des Arbeitszyklus dargestellt ist,
- Fig. 3: die Dialysevorrichtung, wobei der dritte Arbeitstakt des Arbeitszyklus dargestellt ist,
- Fig. 4: die Dialysevorrichtung, wobei der vierte Arbeitstakt des Arbeitszyklus dargestellt ist,
- Fig. 5: die Dialysevorrichtung von Fig. 1, wobei der erste Takt des Arbeitszyklus für den Fall einer Ultrafiltration dargestellt ist,
- Fig. 6: die Dialysevorrichtung von Fig. 5, wobei der zweite Arbeitstakt dargestellt ist,
- Fig. 7: die Dialysevorrichtung, wobei der dritte Arbeitstakt dargestellt ist und
- Fig. 8: die Dialysevorrichtung, wobei der vierte Arbeitstakt dargestellt ist.

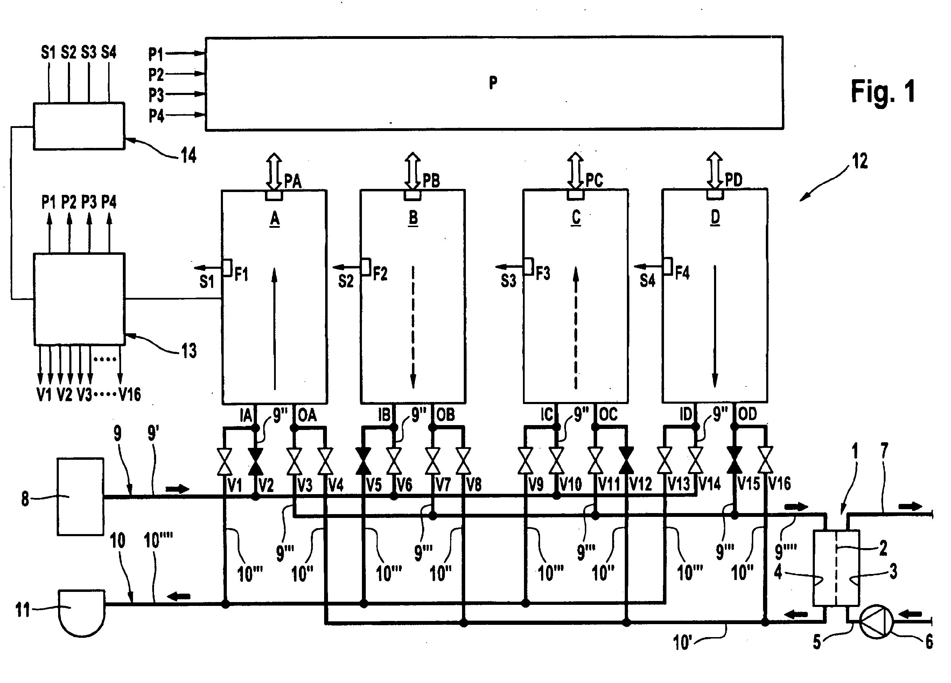

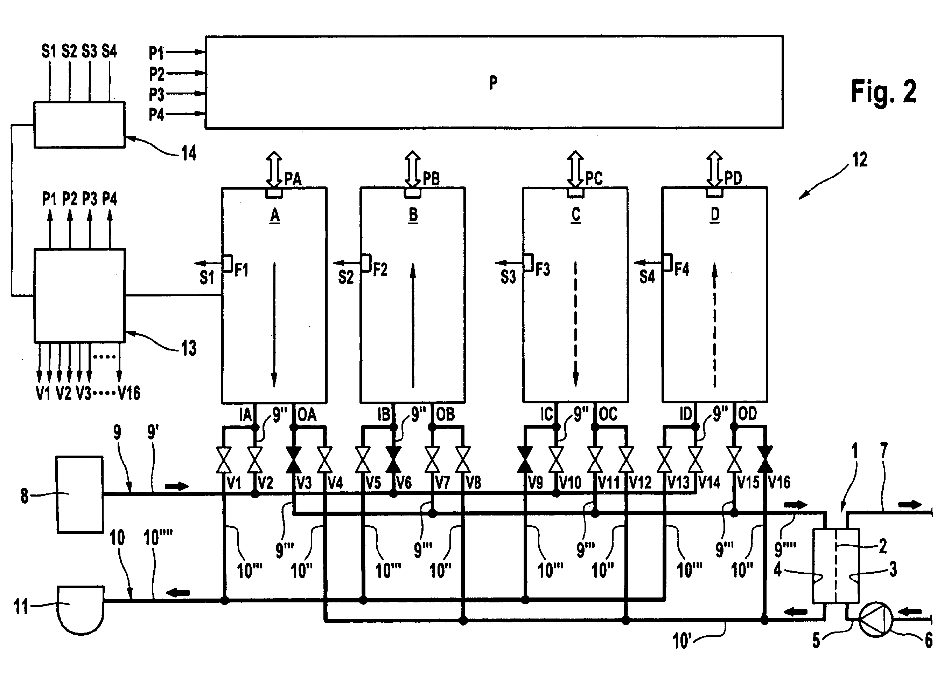

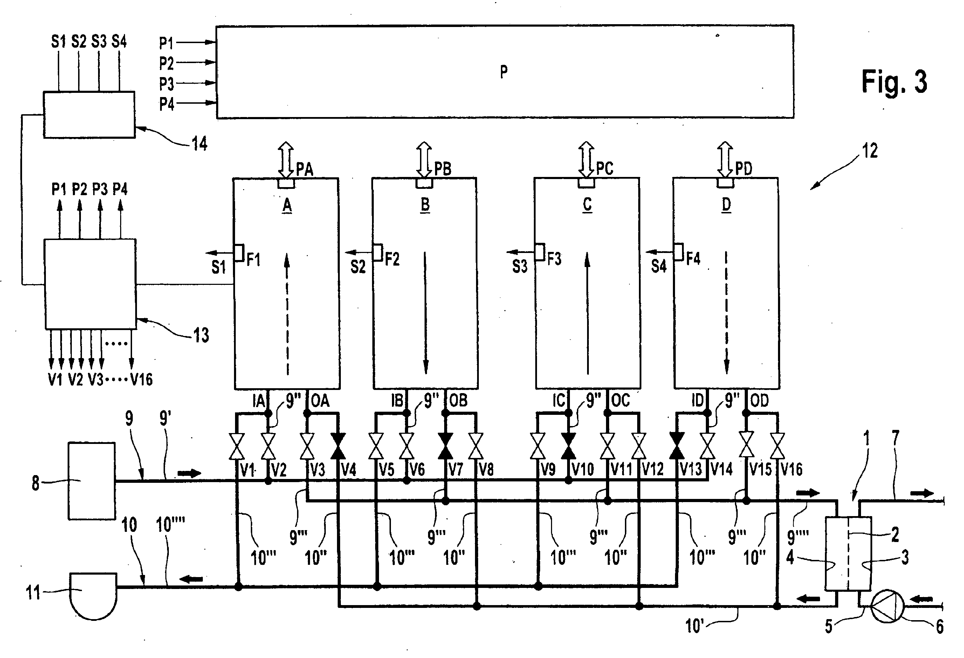

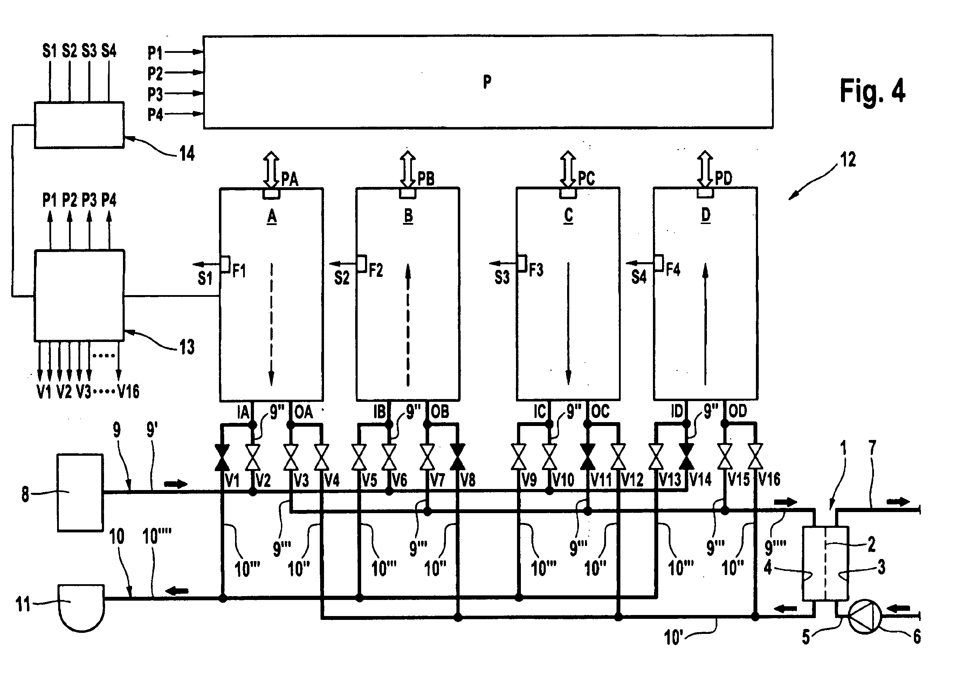

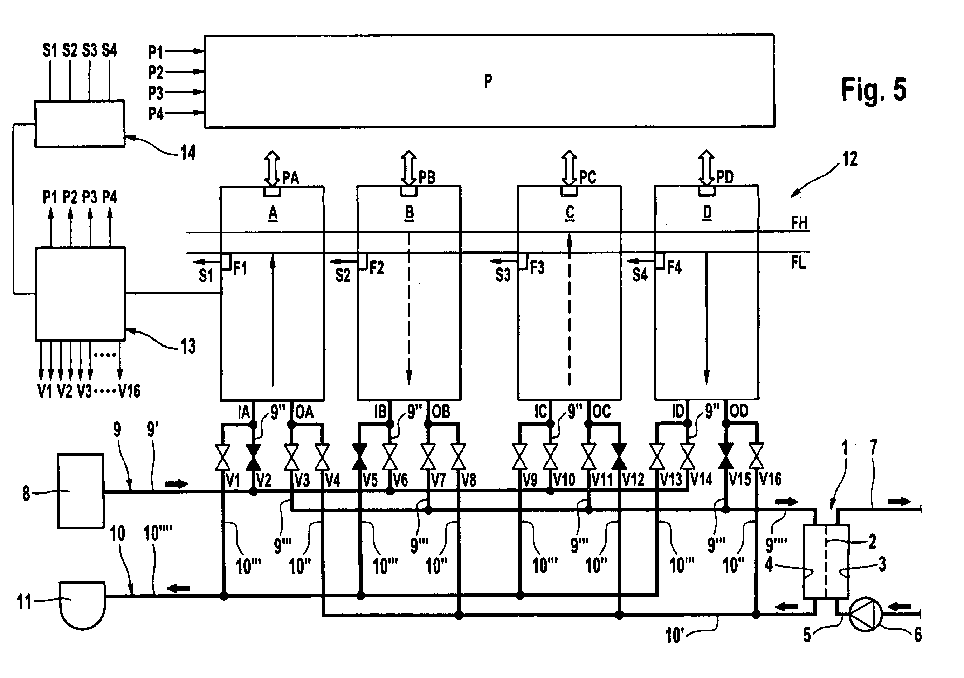

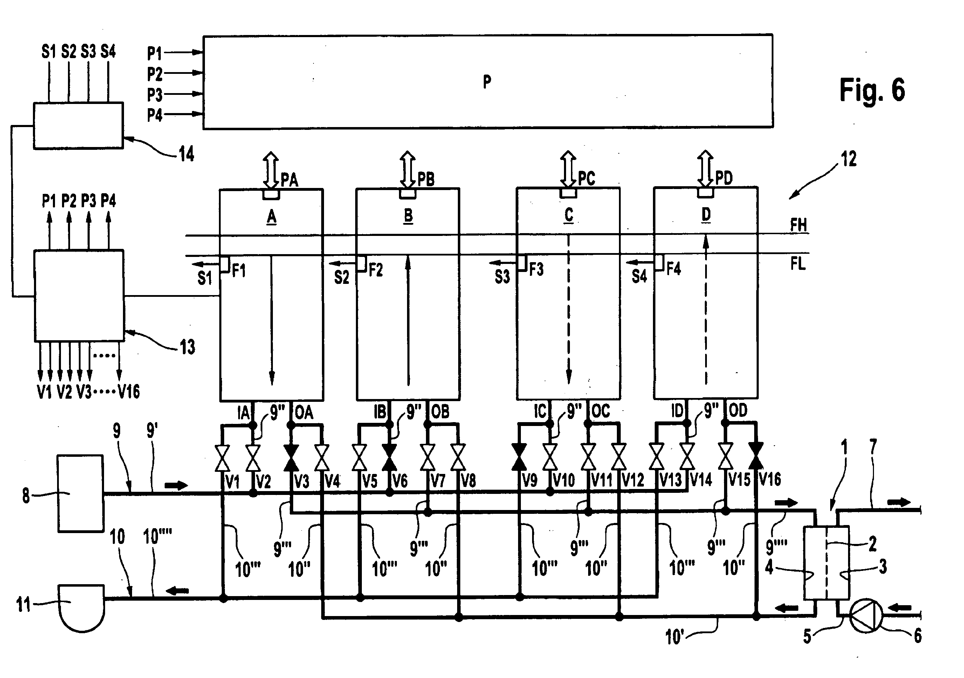

Die Figuren 1-4 zeigen die wesentlichen Komponenten einer Dialysevorrichtung in stark vereinfachter schematischer Darstellung für die einzelnen Arbeitstakte eines vier Arbeitstakte umfassenden Arbeitszyklus für den Fall, dass dem Patienten keine Flüssigkeit entzogen wird. Die Figuren 5-8 zeigen die Arbeitstake der Dialysevorrichtung für den Fall, dass dem Patienten Flüssigkeit entzogen wird. Die einzelnen Komponenten der Dialysevorrichtungen sind in den Figuren mit den gleichen Bezugszeichen versehen.

Die Dialysevorrichtung weist einen Dialysator 1 auf, der durch eine semipermeable Membran 2 in eine Blutkammer 3 und eine Dialysierflüssigkeitskammer 4 unterteilt ist. An dem Einlass der Blutkammer 3 ist eine Blutzuführleitung 5 angeschlossen, in die eine Blutpumpe 6 geschaltet ist. Stromab der Blutkammer führt eine Blutabführleitung 7 von dem Auslass der Blutkammer zu dem Patienten.

In einer Dialysierflüssigkeitsquelle 8 wird frische Dialysierflüssigkeit bereitgestellt. Von der Dialysierflüssigkeitsquelle 8 führt eine Dialysierflüssigkeitszuführleitung 9 zu dem Eingang der Dialysierflüssigkeitskammer 4 des Dialysators, während eine Dialysierflüssigkeitsabführleitung 10 von dem Auslass der Dialysierflüssigkeitskammer des Dialysators zu einem Auslass 11 führt. Zur Bilanzierung von frischer und verbrauchter Dialysierflüssigkeit ist eine Bilanziereinrichtung vorgesehen, die nachfolgend im Einzelnen beschrieben wird.

Die Bilanziereinrichtung 12 umfasst insgesamt vier Bilanzkammern, die mit A, B, C und D bezeichnet sind. Alle Bilanzkammern A, B, C, D haben den gleichen Aufbau. Es handelt sich um Teile eines zur einmaligen Verwendung bestimmten Disposables. Die Bilanzkammern A, B, C, D weisen jeweils einen Einlass bzw. Auslass (Anschluss) IA, IB, IC, ID und einen Einlass bzw. Auslass (Anschluss) OA, OB, OC, OD auf. Darüber hinaus weisen die Bilanzkammern A, B, C, D jeweils einen Anschluss PA, PB, PC, PD auf, an denen nur andeutungsweise dargestellte Mittel P zur Erzeugung eines Überdrucks und/oder Unterdrucks in den Bilanzkammern A, B, C, D angeschlossen sind.

Die Bilanzkammern A, B, C, D sind sowohl in die Dialysierflüssigkeitszuführleitung 9 als auch in die Dialysierflüssigkeitsabführleitung 10 geschaltet, wobei die einzelnen Bilanzkammern mit zugehörigen Absperrorganen in die Flüssigkeitsströmung eingebunden oder von der Flüssigkeitsströmung abgetrennt werden können. Dabei sind jeder Bilanzkammer A, B, C, D jeweils vier Absperrorgane V1 - V16 zugeordnet. Nachfolgend wird die Verschaltung der Bilanzkammern im Einzelnen beschrieben.

Die Dialysierflüssigkeitszuführleitung 9 weist einen ersten Leitungsabschnitt 9' auf, der von der Dialysierflüssigkeitsquelle 8 abgeht. Von dem ersten Leitungsabschnitt 9' zweigen vier Stichleitungen 9"ab, die jeweils zu dem Einlass I der Bilanzkammern A, B, C, D führen. In die Stichleitungen 9" sind die Absperrorgane V2, V6, V10 und V 14 geschaltet. Von dem Auslass der Bilanzkammern A, B, C, D geht jeweils eine Stichleitung 9"' ab, die zu einem zweiten Leitungsabschnit 9"" der Dialysierflüssigkeitszuführleitung 9 führt. In die Stichleitungen 9'" sind die Absperrorgane V3, V7, V11 und V15 geschaltet.

Die Dialysierflüssigkeitsabführleitung 10 weist einen ersten Leitungsabschnitt 10' auf, der von dem Auslass der Dialysierflüssigkeitskammer 4 des Dialysators 1 abgeht. Von dem ersten Leistungsabschnitt 10' zweigen wieder vier Stichleitungen 10" ab, die jeweils zu dem Auslass O der Bilanzkammern A, B, C, D führen. In die Stichleitungen 10" sind die Absperrorgane S4, S8, S12 und S16 geschaltet. Von dem Einlass I der Bilanzkammern A, B, C, D führt jeweils eine Stichleitung 10'" zu einem zweiten Leitungsabschnit 10"" der Dialysierflüssigkeitsabführleitung 10. In die Stichleitungen10"" sind die Absperrorgane V1, V5, V9 und V13 geschaltet.

Darüber hinaus verfügt die Dialysevorrichtung über eine zentrale Steuereinheit 13, die über Steuerleitungen S1- S16 die Absperrorgane V1- V16 öffnet bzw. schließt und über Steuerleitungen P1-P4 die Mittel P zum Erzeugen eines Über- bzw. Unterdrucks derart ansteuert, dass in den Bilanzkammern ein Überdruck bzw. ein Unterdruck erzeugt wird. Die Ansteuerung der Absperrorgane kann elektrisch oder pneumatisch oder hydraulisch erfolgen.

Während des Betriebs der Dialysevorrichtung wird frische Dialysierflüssigkeit, die in der Dialysierflüssigkeitsquelle 8 bereit gestellt wird, gegen verbrauchte Dialysierflüssigkeit bilanziert, die in den Auslass 11 abgeführt wird. Dabei werden die einzelnen Bilanzkammern A, B, C, D derart angesteuert, dass sie mit frischer bzw. verbrauchter Dialysierflüssigkeit befüllt werden bzw. entleert werden. Die Ansteuerung erfolgt in aufeinanderfolgenden Arbeitszyklen, die jeweils vier Arbeitstakte umfassen.

Die Fig. 1-4 zeigen die vier Arbeitstakte eines Arbeitszyklus. Dabei sind die von der Steuereinheit 13 geöffneten Absperrorgane schwarz gekennzeichnet. Ein nach oben weisender Pfeil kennzeichnet das Befüllen einer Bilanzkammer, während ein nach unten weisender Pfeil das Entleeren der Bilanzkammer kennzeichnet. Ein durchgezogener Pfeil kennzeichnet frische Dialysierflüssigkeit, während ein gestrichelter Pfeil verbrauchte Dialysierflüssigkeit kennzeichnet.

In dem ersten Arbeitstakt des Arbeitszyklus öffnet die zentrale Steuereinheit 13 die Absperrorgane V2, V5, V 12 und V15, während die Steuereinheit die anderen Absperrorgane schließt (Fig. 1). Damit lassen sich die vier Bilanzkammern A, B, C, D in ein erstes Paar von Bilanzkammern A,B und ein zweites Paar von Bilanzkammern C, D unterteilen. Der Bilanzkammer A wird aus der Dialysierflüssigkeitsquelle 8 frische Dialysierflüssigkeit zugeführt, während aus der Bilanzkammer B verbrauchte Dialysierflüssigkeit zu dem Auslass 11 abgeführt wird. Aus der Bilanzkammer D wird der Dialysierflüssigkeitskammer 4 des Dialysators 1 frische Dialysierflüssigkeit zugeführt, während verbrauchte Dialysierflüssigkeit aus der Dialysierflüssigkeitskammer 4 des Dialysators 1 in die Bilanzkammer C abgeführt wird. Die entsprechenden Flüssigkeitsströmungen sind in Fig. 1 mit Pfeilen gekennzeichnet. Es wird also der Inhalt der Bilanzkammer D über die Dialysierflüssigkeitskammer des Dialysators in die Bilanzkammer C entleert.

Dabei werden die Bilanzkammern A und C, der Flüssigkeit zugeführt werden, mit einem Unterdruck und die Bilanzkammern B und D, aus der Flüssigkeit abgeführt wird, mit einem Unterdruck beaufschlagt. Hierzu steuert die zentrale Steuereinheit 13 die Mittel P zum Erzeugen eines Über- bzw. Unterdrucks entsprechend an.

Der Vorgang des Befüllens bzw. des Entleerens einer Kammer wird von der zentralen Steuereinheit 13 überwacht. Dabei wirkt die zentrale Steuereinheit 13 mit Mitteln 14 zum Überwachen des Füllstands der einzelnen Bilanzkammern A, B, C, D zusammen, die jeweils über Steuerleitungen S1, S2, S3, S4 den einzelnen Bilanzkammern zugeordnete Füllstandsensoren F1, F2, F3, F4 aufweist. Bei einem vorgegebenen Wert für den Füllstand, der in sämtlichen Kammern gleich ist, werden die entsprechenden Absperrorgane V geschlossen.

In dem zweiten Arbeitstakt bilden die Kammern B und C das erste Paar von Bilanzkammern, während die Kammern A und D das zweite Paar von Bilanzkammern bilden. Der Bilanzkammer B wird frische Dialysierflüssigkeit aus der Dialysierflüssigkeitsquelle 8 zugeführt, während aus der Bilanzkammer C verbrauchte Dialysierflüssigkeit in den Auslass 11 abgeführt wird. Aus der Bilanzkammer A wird der Dialysierflüssigkeitskammer 4 frische Dialysierflüssigkeit zugeführt, während der Bilanzkammer D verbrauchte Dialysierflüssigkeit aus der Dialysierflüssigkeitskammer zugeführt wird. Dabei werden wieder die entsprechenden Absperrorgane V geöffnet und die Bilanzkammern von den Mitteln P zum Erzeugen eines Über- bzw. Unterdrucks wieder entsprechend mit einem Über- oder Unterdruck beaufschlagt.

Im dritten Arbeitstakt bilden die Bilanzkammern C und D das erste Bilanzkammerpaar und die Bilanzkammern A und B das zweite Bilanzkammerpaar. Aus der Dialysierflüssigkeitsquelle 8 strömt frische Dialysierflüssigkeit in die Bilanzkammer C, während verbrauchte Dialysierflüssigkeit aus der Bilanzkammer D in den Auslass 11 strömt. Die frische Dialysierflüssigkeit aus der Bilanzkammer B wird über die Dialysierflüssigkeitskammer 4 in die Bilanzkammer A entleert.

In dem vierten Arbeitstakt bilden die Bilanzkammern A und D das erste Bilanzkammerpaar, während die Bilanzkammern B und C das zweite Bilanzkammerpaar bilden. In die Bilanzkammer D strömt frische Dialysierflüssigkeit, während aus der Bilanzkammer A verbrauchte Dialysierflüssigkeit strömt. Gleichzeitig wird frische Dialysierflüssigkeit aus der Bilanzkammer C über die Dialysierflüssigkeitskammer 4 in die Bilanzkammer B entleert.

Nach dem vierten Arbeitstakt schließt sich wieder der erste Arbeitstakt des folgenden Arbeitszyklus an. Es zeigt sich, dass die Funktionen der einzelnen Bilanzkammern jeweils zyklisch vertauscht werden, wobei jeder Bilanzkammer frische Dialysierflüssigkeit zugeführt und verbrauchte Dialysierflüssigkeit aus jeder Bilanzkammer abgeführt wird.

Das zyklische Vertauschen der Funktion der einzelnen Kammern A, B, C, D bewirkt, dass sich unterschiedliche Füllvolumina, die auf unterschiedliche Kammervolumen oder Fehler bei der Füllstandsmessung zurückgeführt werden können, während eines Arbeitszyklus kompensieren. Dies wird an dem vorliegenden Ausführungsbeispiel nachfolgend erläutert.

Es sei angenommen, dass das Füllvolumen der Bilanzkammer A 100 ml, der Bilanzkammer B 110 ml, der Bilanzkammer C 90 ml und der Bilanzkammer D 105 ml ist. Die Volumina der Schlauchleitungen sollen für das Beispiel vernachlässigt werden. Es sei aber bemerkt, dass die Abweichungen der Volumina der Kammern B, C, D von dem Sollwert von 100 ml unrealistisch hoch sind und nur als Beispiel dienen sollen.

Im ersten Arbeitstakt wird die Bilanzkammer D über die Dialysierflüssigkeitskammer 4 des Dialysators 1 in die Bilanzkammer C entleert. Die Mittel P zum Erzeugen eines Über- oder Unterdrucks, die nachfolgend als Pneumatikeinrichtung P bezeichnet werden, erzeugen in Kammer D einen Überdruck und in Kammer C einen Unterdruck. Es entleeren sich demnach 105 ml aus Kammer D, wobei sich aber bereits nach 90 ml das Absperrorgan V12 im Zulauf der Kammer C schließt. Da Kammer D nicht vollständig entleert ist, bleibt das Absperrorgan V15 im Ablauf dieser Kammer geöffnet und der Überdruck aus Kammer D pflanzt sich in die Dialysierflüssigkeitskammer 4 fort. Dort wird die Restflüssigkeit auf Grund des Druckgradienten durch die semipermeable Membran 2 von der Dialysierflüssigkeitsseite auf die Blutseite gedrückt. Dem Blut des Patienten werden also 15 ml Flüssigkeit zugeführt (Refiltration).

Im zweiten Arbeitstakt entleert sich die Kammer A in Kammer D über die Dialysierflüssigkeitskammer3 . Da Kammer D ein größeres Volumen als Kammer A bei gleicher Füllhöhe hat, bleibt der Unterdruck in Kammer D erhalten. Hierdurch werden über die Membran 2 des Dialysators 15ml Flüssigkeit von der Blutseite auf die Dialysatseite gesaugt (Filtration).

Im dritten Arbeitstakt entleert sich Kammer B in Kammer A. Es kommt zu einer Refiltration von 10 ml Dialysierflüssigkeit. Im vierten Arbeitstakt entleert sich Kammer C in Kammer B. Es kommt zu einer Filtration von 20 ml Dialysierflüssigkeit.

Wenn man nun die Volumenänderung im Patientenblut bilanziert, wird deutlich, dass sich die Abweichungen in den Volumina der Kammern über einen Arbeitszyklus ausgleichen. Es ergibt sich eine Summe von 15 ml - 5 ml + 10 ml -20 ml = 0. Dem Patienten wird also weder Flüssigkeit zugeführt, noch Flüssigkeit entzogen.

Im Folgenden soll verdeutlicht werden, wie ein Bilanzierungsfehler entsteht, wenn die Kammern nicht zyklisch vertauscht werden, d.h. der Arbeitszyklus nur zwei Arbeitstakte umfasst, wobei jeweils ein Paar von Kammern nur frische Dialysierflüssigkeit und das andere Paar von Kammern nur verbrauchte Dialysierflüssigkeit aufnimmt. Im ersten Arbeitstakt würde sich die Kammer D in die Kammer C entleeren. Es käme zu einer Refiltration von 15 ml. Im zweiten Arbeitstakt würde sich die Kammer A in Kammer B entleeren. Es käme zu einer Filtration von 10 ml. Daraus ergäbe sich innerhalb des Arbeitszyklus eine Summe, die 15 ml - 10ml + 15ml - 10ML = 10ml ist. Der Fehler wird also nicht kompensiert.

Die erfindungsgemäße Ansteuerung der Bilanzkammern hat neben der exakten Bilanzierung von frischer und verbrauchter Dialysierflüssigkeit auch den Vorteil, dass für den Fall einer gewünschten Ultrafiltration innerhalb eines Arbeitszyklus die Ultrafiltrationsmenge genau eingestellt werden kann.

Für den Fall der Ultrafiltration gibt die zentrale Steuereinheit 13 zwei unterschiedliche Werte für den Füllstand FH und FL der Kammern vor, wie nachfolgend erläutert wird.

Um Flüssigkeit dem Blut des Patienten zu entziehen (Ultrafiltration) wird der Füllstand der Bilanzkammern, die verbrauchte Dialysierflüssigkeit aus der Dialysierflüssigkeitskammer aufnehmen sollen, so eingestellt, dass deren Füllstand FH über dem Füllstand FL der Bilanzkammern liegt, die mit frischer Dialysierflüssigkeit befüllt sind. Die Differenz beider Füllstände FH - FL entspricht dann der dem Blut des Patienten entzogenen Flüssigkeit. Die Fig. 5-8 zeigen die Arbeitstakte des Arbeitszyklus für denn Fall der Ultrafiltration. Die Fig. 5-8 entsprechen den Fig. 1-4. Allerdings ist in den Fig. 5-8 der unterschiedliche Füllstand FH, FL der Kammern A, B, C, D mit Linien gekennzeichnet. Mit Ausnahme der unterschiedlichen Füllstände der einzelnen Bilanzammern erfolgt die Ansteuerung der Bilanzkammern so, wie unter Bezugnahme auf die Fig. 1-4 beschrieben ist.

Bei dem in den Fig. 5-8 gezeigten Ausführungsbeispiel werden sämtliche Bilanzkammern zur Aufnahme verbrauchter Dialysierflüssigkeit mit einem höheren Füllstand betrieben als die Bilanzkammern, die frische Dialysierflüssigkeit liefern. Es ist aber auch möglich, dass nur eine, zwei oder drei der insgesamt vier Bilanzkammern zur Aufnahme der verbrauchten Dialysierflüssigkeit mit einem höheren Füllstand betrieben werden.

Im ersten Arbeitstakt wird die frische Dialysierflüssigkeit aus der Bilanzkammer D, die in einem vorausgehenden Arbeitstakt bis zu dem Füllstand FL befüllt worden ist, über die Dialysierflüssigkeitskammer 4 des Dialysators 1 in die Bilanzkammer C überführt, wobei die Bilanzkammer C bis zu einem Füllstand FH befüllt wird, der höher als der Füllstand FL ist. Dies wird anhand der Linien in Fig.5 verdeutlicht. Da es sich hier um ein geschlossenes, volumenkonstantes System handelt, wird dem Blut des Patienten über die Membran 2 des Dialysators 1 die Menge an Flüssigkeit entzogen, die der Differenz Δ= FH - FL zwischen den beiden Füllständen entspricht. Im zweiten Arbeitstakt wird die Dialysierflüssigkeit aus der Bilanzkammer A über die Dialysierflüssigkeitskammer in die Bilanzkammer D überführt, wobei dem Blut des Patienten wieder die Menge an Flüssigkeit entzogen wird, die der Differenz Δ= FH - FL der beiden Füllstände entspricht.

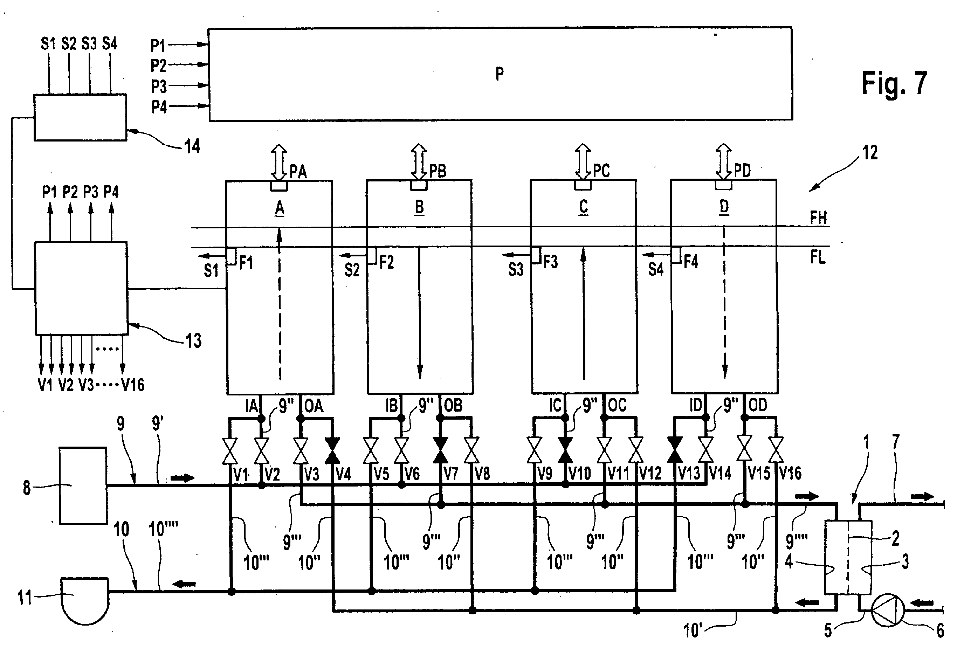

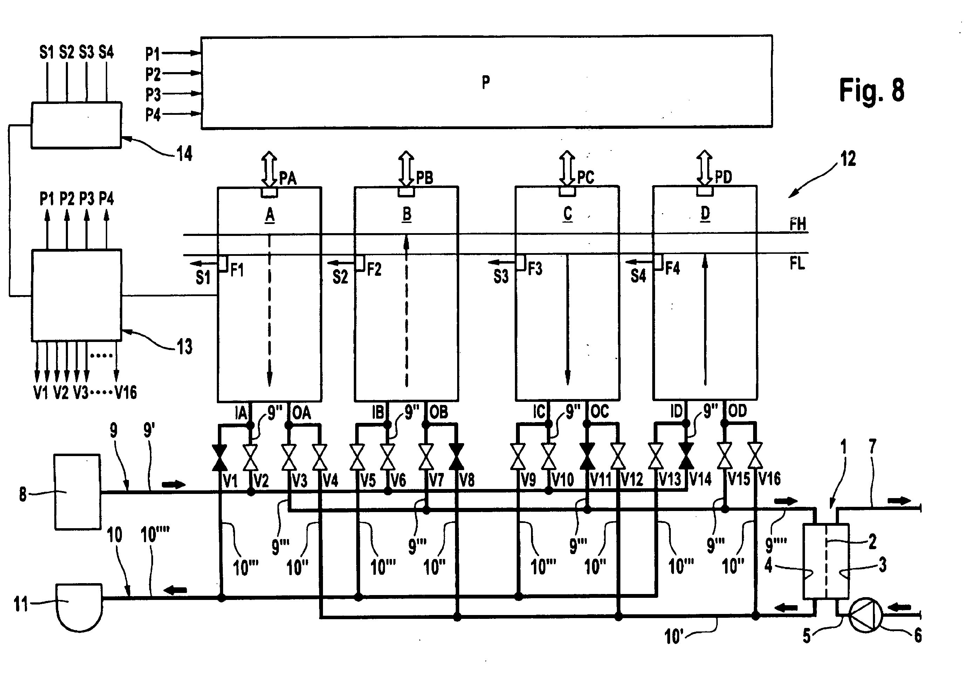

Der dritte und vierte Arbeitstakt (Fig. 7 und 8) entsprechen dem ersten und zweiten Arbeitstakt bei gleichen Füllständen in den Kammern. Auch hier wird in jedem Arbeitstakt dem Patienten eine Menge an Flüssigkeit entzogen, die der Füllstandsdifferenz Δ= FH - FL entspricht. Die Ultrafiltrationsmenge ergibt sich dann aus der Summe sämtlicher Füllstandsdifferenzen in allen Arbeitszyklen über die gesamte Behandlungsdauer. Wegen der vergleichsweise kleinen Füllstandsdifferenzen kann die gewünschte Ultrafiltrationsmenge mit relativ hoher Genauigkeit eingestellt werden.

## Patentansprüche

1. Vorrichtung zur Dialysebehandlung mit
einer Dialysierflüssigkeitsquelle (8) für frische Dialysierflüssigkeit und einem Auslass (11) für verbrauchte Dialysierflüssigkeit,
einem durch eine semipermeable Membran (2) in eine Blutkammer (3) und eine Dialysierflüssigkeitskammer (4) unterteilten Dialysator (1),
Mitteln (12) zum Bilanzieren von frischer und verbrauchter Dialysierflüssigkeit, die eine erste Bilanzkammer (A), eine zweite Bilanzkammer (B), eine dritte Bilanzkammer (C) und eine vierte Bilanzkammer (D) umfassen,
Mitteln (9, 10) zum Leiten von frischer Dialysierflüssigkeit in die Bilanzkammern und Abführen von verbrauchter Dialysierflüssigkeit aus den Bilanzkammern,
Mitteln (P; V1 bis V16) zum Fördern von frischer und verbrauchter Dialysierflüssigkeit, und
eine Steuereinheit (13) zum Ansteuern der Mittel zum Fördern von frischer und verbrauchter Dialysierflüssigkeit,
**dadurch gekennzeichnet,**
**dass** die Mittel (9, 10) zum Leiten von frischer und verbrauchter Dialysierflüssigkeit derart ausgebildet sind, dass
frische Dialysierflüssigkeit aus der Dialysierflüssigkeitsquelle (8) jeder Bilanzkammer (A, B, C, D) zugeführt werden kann und frische Dialysierflüssigkeit aus jeder Bilanzkammer (A, B, C, D) der Dialysierflüssigkeitskammer (4) des Dialysators (1) zugeführt werden kann, und
verbrauchte Dialysierflüssigkeit aus der Dialysierflüssigkeitskammer (4) des Dialysators (1) jeder Bilanzkammer (A, B, C, D) zugeführt werden kann und verbrauchte Dialysierflüssigkeit aus jeder Bilanzkammer abgeführt werden kann, und
**dass** die Steuereinheit (13) mit den Mitteln (P; V1 bis V16) zum Fördern von frischer und verbrauchter Dialysierflüssigkeit derart zusammenwirkt, dass in aufeinanderfolgenden Arbeitstakten aus einer Bilanzkammer von einem Paar von Bilanzkammern der Dialysierflüssigkeitskammer frische Dialysierflüssigkeit zugeführt und der anderen Bilanzkammer von dem Paar von Bilanzkammern verbrauchte Dialysierflüssigkeit aus der Dialysierflüssigkeitskammer zugeführt wird, während einer Bilanzkammer von einem anderen Paar von Bilanzkammern von der Dialysierflüssigkeitsquelle frische Dialysierflüssigkeit zugeführt und aus der anderen Bilanzkammer von dem anderen Paar von Bilanzkammern verbrauchte Dialysierflüssigkeit abgeführt wird.

2. Vorrichtung nach Anspruch 1, **dadurch gekennzeichnet, dass** die Mittel (P; V1 bis V 16) zum Fördern von Dialysierflüssigkeit Mittel (P) zum Beaufschlagen der Bilanzkammern mit einem Überdruck oder Unterdruck und Mittel (V1 bis V16) zum Öffnen und Absperren der Mittel (9, 10) zum Leiten von Dialysierflüssigkeit aufweisen.

3. Vorrichtung nach Anspruch 2, **dadurch gekennzeichnet, dass** die Mittel zum Fördern (P; V1 bis V16) von Dialysierflüssigkeit Mittel (F1 bis F4) zum Messen des Füllstandes der Bilanzkammern aufweisen, wobei die Steuereinheit (13) derart ausgebildet ist, dass die Mittel (VI bis V16) zum Öffnen und Absperren in Abhängigkeit von einem vorgegebenen Wert (FH, FL) für den Füllstand in den Bilanzkammern geöffnet oder geschlossen werden.

4. Vorrichtung nach Anspruch 3, **dadurch gekennzeichnet, dass** die Steuereinheit (13) derart ausgebildet ist, dass ein erster Wert (FL) für den Füllstand der Bilanzkammer von dem einen Paar von Bilanzkammern, von der frische Dialysierflüssigkeit der Dialysierflüssigkeitskammer zugeführt wird, und ein zweiter Wert (FH) für den Füllstand der anderen Bilanzkammer von dem einen Paar von Bilanzkammern, der verbrauchte Dialysierflüssigkeit aus der Dialysierflüssigkeitskammer zugeführt wird, vorgegeben wird, wobei der erste Wert von dem zweiten Wert verschieden ist.

5. Vorrichtung nach Anspruch 3 oder 4, **dadurch gekennzeichnet, dass** die Steuereinheit (13) derart ausgebildet ist, dass ein erster Wert (FL) für den Füllstand der Bilanzkammer von dem anderen Paar von Bilanzkammem, der frische Dialysierflüssigkeit von der Dialysierflüssigkeitsquelle zugeführt wird, und ein zweiter Wert (FH) für den Füllstand der anderen Bilanzkammer von dem anderen Paar von Bilanzkammern, von der verbrauchte Dialysierflüssigkeit aus der Dialysierflüssigkeitskammer abgeführt wird, vorgegeben wird, wobei der erste Wert von dem zweiten Wert verschieden ist.

6. Vorrichtung nach Anspruch 4 oder 5, **dadurch gekennzeichnet, dass** der erste Wert (FL) für den Füllstand kleiner als der zweite Wert (FH) für den Füllstand ist.

7. Vorrichtung nach einem der Ansprüche 1 bis 6, **dadurch gekennzeichnet, dass** die Mittel (9, 10) zum Leiten von Dialysierflüssigkeit eine Zuführleitung (9) zum Zuführen von frischer Dialysierflüssigkeit aus der Dialysierflüssigkeitsquelle (8) zu dem Einlass der Dialysierflüssigkeitskammer (4) aufweisen, wobei die erste Bilanzkammer (A), die zweite Bilanzkammer (B), die dritte Bilanzkammer (C) und die vierte Bilanzkammer (D) in die Zuführleitung geschaltet sind, und eine Abführleitung (10) zum Abführen von verbrauchter Dialysierflüssigkeit von der Dialysierflüssigkeitskammer (4) aufweisen, wobei die erste Bilanzkammer (A), die zweite Bilanzkammer (B), die dritte Bilanzkammer (C) und die vierte Bilanzkammer (D) in die Abführleitung geschaltet sind.

8. Vorrichtung nach Anspruch 7, **dadurch gekennzeichnet, dass** von der ersten Kammer (A), der zweiten Kammer (B), der dritten Kammer (C) und der vierten Kammer (D) zu der Zuführleitung (9) führende Stichleitungen (9") und zu der Abführleitung (10) führende Stichleitungen (10") abzweigen, wobei die Mittel (V1 bis V16) zum Öffnen und Absperren in den Stichleitungen angeordnete Absperrorgane sind.

9. Vorrichtung nach einem der Ansprüche 1 bis 8, **dadurch gekennzeichnet, dass** die Bilanzkammern (A, B, C, D) und die Mittel (9, 10) zum Leiten von frischer und verbrauchter Dialysierflüssigkeit als Disposable ausgebildet sind.

10. Verfahren zum Bilanzieren von frischer und verbrauchter Dialysierflüssigkeit mit einer Bilanziereinrichtung, die eine erste Bilanzkammer, eine zweite Bilanzkammer, eine dritte Bilanzkammer und eine vierte Bilanzkammer aufweist, **dadurch gekennzeichnet, dass**
die Bilanzkammern (A, B, C, D) derart betrieben werden, dass in aufeinanderfolgenden Arbeitstakten jeder Bilanzkammer der ersten, zweiten, dritten und vierten Bilanzkammer frische Dialysierflüssigkeit zugeführt wird, aus jeder Bilanzkammer frische Dialysierflüssigkeit einer Dialysierflüssigkeitskammer eines Dialysators zugeführt wird, jeder Bilanzkammer verbrauchte Dialysierflüssigkeit aus der Dialysierflüssigkeitskammer des Dialysators zugeführt wird und aus jeder Bilanzkammer verbrauchte Dialysierflüssigkeit abgeführt wird.

11. Verfahren nach Anspruch 10, **dadurch gekennzeichnet, dass** in aufeinanderfolgenden Arbeitstakten aus einer Bilanzkammer von einem Paar von Bilanzkammern der Dialysierflüssigkeitskammer frische Dialysierflüssigkeit zugeführt und der anderen Bilanzkammer von dem Paar von Bilanzkammern verbrauchte Dialysierflüssigkeit aus der Dialysierflüssigkeitskammer zugeführt wird, während einer Bilanzkammer von einem anderen Paar von Bilanzkammern von der Dialysierflüssigkeitsquelle frische Dialysierflüssigkeit zugeführt und aus der anderen Bilanzkammer von dem anderen Paar von Bilanzkammern verbrauchte Dialysierflüssigkeit abgeführt wird.

12. Verfahren nach Anspruch 10 oder 11, **dadurch gekennzeichnet, dass** zum Fördern von Dialysierflüssigkeit aus den Bilanzkammern ein Überdruck in den Bilanzkammern erzeugt wird und zum Fördern von Dialysierflüssigkeit aus den Bilanzkammern in den Bilanzkammern ein Unterdruck erzeugt wird.

13. Verfahren nach einem der Ansprüche 10 bis 12, **dadurch gekennzeichnet, dass** der Füllstand (FL, FH) der Bilanzkammern überwacht wird, wobei die Flüssigkeitsweg in die bzw. aus den Bilanzkammern in Abhängigkeit von einem vorgegebenen Wert für den Füllstand in den Bilanzkammern geöffnet oder geschlossen wird.

14. Verfahren nach Anspruch 13, **dadurch gekennzeichnet, dass** ein erster Wert für den Füllstand (FL) der Bilanzkammer von dem einen Paar von Bilanzkammern, von der frische Dialysierflüssigkeit der Dialysierflüssigkeistkammer zugeführt wird, und ein zweiter Wert (FH) für den Füllstand der anderen Bilanzkammer von dem einen Paar von Bilanzkammern, der verbrauchte Dialysierflüssigkeit aus der Dialysierflüssigkeitskammer zugeführt wird, vorgegeben wird, wobei der erste Wert von dem zweiten Wert verschieden ist.

15. Verfahren nach Anspruch 13 oder 14, **dadurch gekennzeichnet, dass** ein erster Wert für den Füllstand der Bilanzkammer von dem anderen Paar von Bilanzkammern, der frische Dialysierflüssigkeit von der Dialysierflüssigkeitsquelle zugeführt wird, und ein zweiter Wert für den Füllstand der anderen Bilanzkammer von dem anderen Paar von Bilanzkammern, von der verbrauchte Dialysierflüssigkeit aus der Dialysierflüssigkeitskammer abgeführt wird, vorgegeben wird, wobei der erste Wert von dem zweiten Wert verschieden ist.

16. Verfahren nach Anspruch 15, **dadurch gekennzeichnet, dass** der erste Wert (FL) für den Füllstand kleiner als der zweite Wert (FH) für den Füllstand ist.

## Claims

1. Apparatus for dialysis treatment comprising:
a dialysis fluid source (8) for fresh dialysis fluid and a discharge (11) for used dialysis fluid,
a dialyser (1) which is divided by a semi-permeable membrane (2) into a blood chamber (3) and a dialysis fluid chamber (4),
means (12) for balancing fresh and used dialysis fluid, comprising a first balancing chamber (A), a second balancing chamber (B), a third balancing chamber (C) and a fourth balancing chamber (D),
means (9, 10) for conducting fresh dialysis fluid into the balancing chambers and for taking used dialysis fluid away from the balancing chambers,
means (P; V1 to V16) for conveying fresh and used dialysis fluid; and
a control unit (13) for controlling the means for feeding fresh and used dialysis fluid,
**characterised in that**
the means (9, 10) for conducting fresh and used dialysis fluid are configured such that fresh dialysis fluid can be fed from the dialysis fluid source (8) to each balancing chamber (A, B, C, D) and fresh dialysis fluid can be fed from each balancing chamber (A, B, C, D) to the dialysis fluid chamber (4) of the dialyser (1), and
used dialysis fluid can be fed from the dialysis fluid chamber (4) of the dialyser (1) to each balancing chamber (A, B, C, D) and used dialysis fluid can be taken away from each balancing chamber, and
**in that** the control unit (13) cooperates with the means (P; V1 to V16) for conveying fresh and used dialysis fluid in such a way that, in successive phases of operation, fresh dialysis fluid is fed to the dialysis fluid chamber from one balancing chamber of one pair of balancing chambers and used dialysis fluid is fed from the dialysis fluid chamber to the other balancing chamber of the pair of balancing chambers, while fresh dialysis fluid is fed from the dialysis fluid source to one balancing chamber of another pair of balancing chambers and used dialysis fluid is taken away from the other balancing chamber of the other pair of balancing chambers.

2. Apparatus according to claim 1, **characterised in that** the means (P; V1 to V16) for conveying dialysis fluid comprise means (P) for applying excess pressure or a vacuum to the balancing chambers, and means (V1 to V16) for opening and shutting off the means (9, 10) for conducting dialysis fluid.

3. Apparatus according to claim 2, **characterised in that** the means for conveying (P; V1 to V16) dialysis fluid comprise means (F1 to F4) for measuring the fill level of the balancing chambers, the control unit (13) being configured such that the means (V1 to V16) for opening and shutting off are opened and closed depending on a preset value (FH, FL) for the fill level in the balancing chambers.

4. Apparatus according to claim 3, **characterised in that** the control unit (13) is configured such that a first value (FL) is preset for the fill level of the balancing chamber of the one pair of balancing chambers, from which fresh dialysis fluid is fed to the dialysis fluid chamber, and a second value (FH) is preset for the fill level of the other balancing chamber of the one pair of balancing chambers, to which used dialysis fluid is fed from the dialysis fluid chamber, the first value being different from the second value.

5. Apparatus according to either claim 3 or claim 4, **characterised in that** the control unit (13) is configured such that a first value (FL) is preset for the fill level of the balancing chamber of the other pair of balancing chambers, to which fresh dialysis fluid is fed from the dialysis fluid source, and a second value (FH) is preset for the fill level of the other balancing chamber of the other pair of balancing chambers, from which used dialysis fluid is taken away from the dialysis fluid chamber, the first value being different from the second value.

6. Apparatus according to either claim 4 of claim 5, **characterised in that** the first fill level value (FL) is lower than the second fill level value (FH).

7. Apparatus according to any of claims 1 to 6, **characterised in that** the means (9, 10) for conducting dialysis fluid comprise a feed line (9) for feeding fresh dialysis fluid from the dialysis fluid source (8) to the inlet of the dialysis fluid chamber (4), the first balancing chamber (A), the second balancing chamber (B), the third balancing chamber (C) and the fourth balancing chamber (D) each being connected into the feed line, and a takeaway line (10) for taking used dialysis fluid away from the dialysis fluid chamber (4), the first balancing chamber (A), the second balancing chamber (B), the third balancing chamber (C) and the fourth balancing chamber (D) each being connected into the takeaway line.

8. Apparatus according to claim 7, **characterised in that** branch lines (9") which run to the feed line (9) and branch lines (10") which run to the takeaway line (10) branch off from the first chamber (A), the second chamber (B), the third chamber (C) and the fourth chamber (D),
the means (V1 to V16) for opening and shutting off being shut-off members which are arranged in the branch lines.

9. Apparatus according to any of claims 1 to 8, **characterised in that** the balancing chambers (A, B, C, D) and the means (9, 10) for conducting fresh and used dialysis fluid are in the form of disposable items.

10. Method for balancing fresh and used dialysis fluid using a balancing device which comprises a first balancing chamber, a second balancing chamber, a third balancing chamber and a fourth balancing chamber,
**characterised in that**
the balancing chambers (A, B, C, D) are operated in such a way that, in successive phases of operation, fresh dialysis fluid is fed to each of the first, second, third and fourth balancing chambers, fresh dialysis fluid is fed from each balancing chamber to a dialysis fluid chamber of a dialyser, used dialysis fluid is fed to each balancing chamber from the dialysis fluid chamber of the dialyser, and used dialysis fluid is taken away from each balancing chamber.

11. Method according to claim 10, **characterised in that**, in successive phases of operation, fresh dialysis fluid is fed to the dialysis fluid chamber from one balancing chamber of one pair of balancing chambers and used dialysis fluid is fed from the dialysis fluid chamber to the other balancing chamber of the pair of balancing chambers, while fresh dialysis fluid is fed from the dialysis fluid source to one balancing chamber of another pair of balancing chambers and used dialysis fluid is taken away from the other balancing chamber of the other pair of balancing chambers.

12. Method according to either claim 10 or claim 11, **characterised in that** excess pressure is generated in the balancing chambers to convey dialysis fluid out of the balancing chambers and a vacuum is generated in the balancing chambers to convey dialysis fluid out of the balancing chambers.

13. Method according to any of claims 10 to 12, **characterised in that** the fill level (FL, FH) of the balancing chambers is monitored, the fluid path into or out of the balancing chambers being opened or closed depending on a preset value for the fill level in the balancing chambers.

14. Method according to claim 13, **characterised in that** a first value is preset for the fill level (FL)of the balancing chamber of the one pair of balancing chambers, from which fresh dialysis fluid is fed to the dialysis fluid chamber, and a second value (FH) is preset for the fill level of the other balancing chamber of the one pair of balancing chambers, to which used dialysis fluid is fed from the dialysis fluid chamber, the first value being different from the second value.

15. Method according to either claim 13 or claim 14, **characterised in that** a first value is preset for the fill level of the balancing chamber of the other pair of balancing chambers, to which fresh dialysis fluid is fed from the dialysis fluid source, and a second value is preset for the fill level of the other balancing chamber of the other pair of balancing chambers, from which used dialysis fluid is taken away from the dialysis fluid chamber, the first value being different from the second value.

16. Method according to claim 15, **characterised in that** the first fill level value (FL) is less than the second fill level value (FH).

## Revendications

1. Dispositif pour un traitement par dialyse, comprenant
une source de dialysat (8) pour le dialysat frais et une sortie (11) pour le dialysat usé,
un dialyseur (1) divisé par une membrane (2) semi-perméable en une chambre pour le sang (3) et une chambre pour le dialysat (4),
des moyens (12) destinés à évaluer le dialysat frais et le dialysat usé, lesquels comprennent une première chambre d'évaluation (A), une deuxième chambre d'évaluation (B), une troisième chambre d'évaluation (C) et une quatrième chambre d'évaluation (D),
des moyens (9, 10) destinés à guider le dialysat frais dans les chambres d'évaluation et à évacuer le dialysat usé hors des chambres d'évaluation,
des moyens (P ; V1 à V16) destinés à transporter le dialysat frais et le dialysat usé, et
une unité de commande (13) permettant de commander les moyens destinés à transporter le dialysat frais et le dialysat usé,
**caractérisé**
**en ce que** les moyens (9, 10) destinés à guider le dialysat frais et le dialysat usé sont configurés de telle sorte
que le dialysat frais peut être acheminé à partir de la source de dialysat (8) vers chaque chambre d'évaluation (A, B, C, D) et que le dialysat frais peut être acheminé à partir de chaque chambre d'évaluation (A, B, C, D) vers la chambre pour dialysat (4) du dialyseur (1), et que
le dialysat usé peut être acheminé hors de la chambre pour dialysat (4) du dialyseur (1) vers chaque chambre d'évaluation (A, B, C, D) et que le dialysat usé peut être évacué hors de chaque chambre d'évaluation, et
**en ce que** l'unité de commande (13) coopère avec les moyens (P ; V1 à V16) destinés à transporter le dialysat frais et le dialysat usé, de telle sorte que dans des cycles de travail successifs, le dialysat frais est acheminé à partir d'une chambre d'évaluation d'une paire de chambres d'évaluation vers la chambre pour dialysat, et le dialysat usé est acheminé vers l'autre chambre d'évaluation d'une paire de chambres d'évaluation en sortant de la chambre pour dialysat, tandis que le dialysat frais est acheminé vers une chambre d'évaluation d'une autre paire de chambres d'évaluation en sortant de la source de dialysat, et le dialysat usé est évacué hors de l'autre chambre d'évaluation de l'autre paire de chambres d'évaluation.

2. Dispositif selon la revendication 1, **caractérisé en ce que** les moyens (P ; V1 à V16) destinés à transporter le dialysat comprennent des moyens (P) destinés à solliciter les chambres d'évaluation avec une surpression ou une dépression et des moyens (V1 à V16) destinés à ouvrir et fermer les moyens (9, 10) destinés à guider le dialysat.

3. Dispositif selon la revendication 2, **caractérisé en ce que** les moyens (P ; V1 à V16) destinés à transporter le dialysat comprennent des moyens (F1 à F4) destinés à mesurer le niveau de remplissage des chambres d'évaluation, l'unité de commande (13) étant configurée de telle sorte que les moyens (V1 à V16) destinés à ouvrir et fermer sont ouverts ou fermés en fonction d'une valeur (FH, FL) prédéfinie pour le niveau de remplissage dans les chambres d'évaluation.

4. Dispositif selon la revendication 3, **caractérisé en ce que** l'unité de commande (13) est configurée de telle sorte que sont prédéfinies une première valeur (FL) pour le niveau de remplissage de la chambre d'évaluation de l'une des paires de chambres d'évaluation, à partir de laquelle le dialysat frais est acheminé vers la chambre pour dialysat, et une deuxième valeur (FH) pour le niveau de remplissage de l'autre chambre d'évaluation de l'une des paires de chambres d'évaluation, vers laquelle est acheminé le dialysat usé sortant de la chambre pour dialysat, la première valeur étant différente de la deuxième valeur.

5. Dispositif selon la revendication 3 ou 4, **caractérisé en ce que** l'unité de commande (13) est configurée de telle sorte que sont prédéfinies une première valeur (FL) pour le niveau de remplissage de la chambre d'évaluation de l'autre paire de chambres d'évaluation, vers laquelle est acheminé le dialysat frais sortant de la source de dialysat, et une deuxième valeur (FH) pour le niveau de remplissage de l'autre chambre d'évaluation de l'autre paire de chambres d'évaluation, à partir de laquelle est évacué le dialysat usé sortant de la chambre pour dialysat, la première valeur étant différente de la deuxième valeur.

6. Dispositif selon la revendication 4 ou 5, **caractérisé en ce que** la première valeur (FL) pour le niveau de remplissage est inférieure à la deuxième valeur (FH) pour le niveau de remplissage.

7. Dispositif selon l'une quelconque des revendications 1 à 6, **caractérisé en ce que** les moyens (9, 10) destinés à guider le dialysat comprennent une conduite d'alimentation (9) destinée à guider le dialysat frais à partir de la source de dialysat (8) vers l'entrée de la chambre pour dialysat (4), la première chambre d'évaluation (A), la deuxième chambre d'évaluation (B), la troisième chambre d'évaluation (C) et la quatrième chambre d'évaluation (D) étant reliées à ladite conduite d'alimentation, et une conduite d'évacuation (10) destinée à évacuer le dialysat usé à partir de la chambre pour dialysat (4), la première chambre d'évaluation (A), la deuxième chambre d'évaluation (B), la troisième chambre d'évaluation (C) et la quatrième chambre d'évaluation (D) étant reliée à ladite conduite d'évacuation.

8. Dispositif selon la revendication 7, **caractérisé en ce qu'**à partir de la première chambre d'évaluation (A), la deuxième chambre d'évaluation (B), la troisième chambre d'évaluation (C) et la quatrième chambre d'évaluation (D) partent des conduites de dérivation (9") menant vers la conduite d'alimentation (9) et des conduites de dérivation (10") menant vers la conduite d'évacuation (10), les moyens (V1 à V16) destinés à ouvrir et fermer étant des organes de fermeture montés dans lesdites conduites de dérivation.

9. Dispositif selon l'une quelconque des revendications 1 à 8, **caractérisé en ce que** les chambres d'évaluation (A, B, C, D) et les moyens (9, 10) destinés à guider le dialysat frais et le dialysat usé sont réalisés sous forme de dispositifs jetables.

10. Procédé permettant d'évaluer le dialysat frais et le dialysat usé au moyen d'un dispositif d'évaluation qui comprend une première chambre d'évaluation, une deuxième chambre d'évaluation, une troisième chambre d'évaluation et une quatrième chambre d'évaluation,
**caractérisé en ce que**
les chambres d'évaluation (A, B, C, D) sont exploitées de telle sorte que dans des cycles de travail successifs, le dialysat frais est acheminé vers chacune des chambres d'évaluation, à savoir la première chambre d'évaluation, la deuxième chambre d'évaluation, la troisième chambre d'évaluation et la quatrième chambre d'évaluation, le dialysat frais est acheminé à partir de chaque chambre d'évaluation vers une chambre pour dialysat d'un dialyseur, un dialysat usé sortant de la chambre pour dialysat du dialyseur est acheminé vers chaque chambre d'évaluation et le dialysat usé est évacué hors de chaque chambre d'évaluation.

11. Procédé selon la revendication 10, **caractérisé en ce que** dans des cycles de travail successifs, le dialysat frais est acheminé à partir d'une chambre d'évaluation d'une paire de chambres d'évaluation vers la chambre pour dialysat, et le dialysat usé est acheminé vers l'autre chambre d'évaluation de la paire de chambres d'évaluation en sortant de la chambre pour dialysat, tandis que le dialysat frais est acheminé vers une chambre d'évaluation d'une autre paire de chambres d'évaluation en sortant de la source de dialysat, et le dialysat usé est évacué hors de l'autre chambre d'évaluation de l'autre paire de chambres d'évaluation.

12. Procédé selon la revendication 10 ou 11, **caractérisé en ce que** pour transporter le dialysat hors des chambres d'évaluation, une surpression est générée dans les chambres d'évaluation et pour transporter le dialysat hors des chambres d'évaluation, une dépression est générée dans les chambres d'évaluation.

13. Procédé selon l'une quelconque des revendications 10 à 12, **caractérisé en ce que** le niveau de remplissage (FL, FH) des chambres d'évaluation est contrôlé, la voie du liquide vers les ou hors des chambres d'évaluation étant ouverte ou fermée en fonction d'une valeur prédéfinie du niveau de remplissage dans les chambres d'évaluation.

14. Procédé selon la revendication 13, **caractérisé en ce que** sont prédéfinies une première valeur pour le niveau de remplissage (FL) de la chambre d'évaluation de l'une des paires de chambres d'évaluation, à partir de laquelle le dialysat frais est acheminé vers la chambre pour dialysat, et une deuxième valeur (FH) pour le niveau de remplissage de l'autre chambre d'évaluation de l'une des paires de chambres d'évaluation, vers laquelle est acheminé le dialysat usé sortant de la chambre pour dialysat, la première valeur étant différente de la deuxième valeur.

15. Procédé selon la revendication 13 ou 14, **caractérisé en ce que** sont prédéfinies une première valeur pour le niveau de remplissage de la chambre d'évaluation de l'autre paire de chambres d'évaluation, vers laquelle est acheminé le dialysat frais sortant de la source de dialysat, et une deuxième valeur pour le niveau de remplissage de l'autre chambre d'évaluation de l'autre paire de chambres d'évaluation, à partir de laquelle est évacué le dialysat usé sortant de la chambre pour dialysat, la première valeur étant différente de la deuxième valeur.

16. Procédé selon la revendication 15, **caractérisé en ce que** la première valeur (FL) pour le niveau de remplissage est inférieure à la deuxième valeur (FH) pour le niveau de remplissage.
